(19) Europäisches Patentamt
European Patent Office
Office européen des brevets

(11) EP 2 494 995 A2

(12) EUROPEAN PATENT APPLICATION

(43) Date of publication:
**05.09.2012 Bulletin 2012/36**

(51) Int Cl.:
***A61L 15/44*** *(2006.01)* ***A61K 9/70*** *(2006.01)*

(21) Application number: **12153805.2**

(22) Date of filing: **03.02.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **03.03.2011 JP 2011046095**

(71) Applicant: **Nitto Denko Corporation
Ibaraki-shi, Osaka 567-8680 (JP)**

(72) Inventors:
- **Hashino, Ryo
Osaka, 567-8680 (JP)**
- **Kuroda, Hidetoshi
Osaka, 567-8680 (JP)**
- **Ameyama, Satoshi
Osaka, 567-8680 (JP)**
- **Satoda, Shiro
Osaka, 567-8680 (JP)**
- **Ishikura, Jun
Osaka, 567-8680 (JP)**

(74) Representative: **HOFFMANN EITLE
Patent- und Rechtsanwälte
Arabellastrasse 4
81925 München (DE)**

(54) **Patch and patch preparation**

(57) A patch according to an embodiment of the present invention includes a support and a pressure-sensitive adhesive layer provided on at least one surface of the support. The pressure-sensitive adhesive layer contains an acrylic copolymer obtained by copolymerizing a monomer mixture containing (a) a (meth)acrylic acid alkyl ester, (b) a hydroxyl group-containing monomer, and (c) a diketone group-containing monomer.

EP 2 494 995 A2

## Description

### BACKGROUND OF THE INVENTION

#### 1. Field of the Invention

The present invention relates to a patch having a support and a pressure-sensitive adhesive layer on at least one surface of the support, and a patch preparation obtained by incorporating a drug into the pressure-sensitive adhesive layer of such patch.

#### 2. Description of the Related Art

A patch and a patch preparation each of which is used by being attached to a skin for the purpose of, for example, protecting the skin or transdermally administering a drug are each requested to show sufficient pressure-sensitive adhesiveness upon attachment to the skin and to be capable of being released and removed after its use without contaminating the surface of the skin (for example, causing an adhesive residue, stickiness, or the like). In addition, it is desirable that the patch and the patch preparation be lowly stimulant to the skin.

International Patent WO2006/064747A describes a patch using a pressure-sensitive adhesive obtained by blending a copolymer A, which is obtained by copolymerizing methyl methacrylate and diacetone acrylamide, and a copolymer B, which is obtained by copolymerizing a monomer mixture containing 2-hydroxyethyl acrylate. However, the patch using such pressure-sensitive adhesive may cause stringing at the time of its release from a skin depending on conditions.

Japanese Patent Application Laid-open No. 2005-15536 describes a patch using a pressure-sensitive adhesive containing a copolymer obtained by copolymerizing methyl methacrylate and 2-acetoacetoxyethyl methacrylate. However, the patch may also cause stringing at the time of its release from a skin depending on conditions.

### SUMMARY OF THE INVENTION

The present invention has been made to solve the above-mentioned conventional problems, and an object of the present invention is to provide a patch that has a remarkably excellent cohesive strength, causes no stringing at the time of its release from a skin, and can realize a patch preparation having excellent stability.

The inventors of the present invention have made extensive studies about the relationship between composition of a material constituting a pressure-sensitive adhesive layer of a patch and characteristics of the pressure-sensitive adhesive layer. As a result, the inventors have found that the use of an acrylic copolymer obtained by copolymerizing a monomer mixture containing a (meth)acrylic acid alkylester, a hydroxyl group-containing-monomer, and a diketone group-containing monomer can achieve the above-mentioned object. Thus, the inventors have completed the present invention.

According to one aspect of the present invention, a patch is provided. The patch includes a support and a pressure-sensitive adhesive layer provided on at least one surface of the support. The pressure-sensitive adhesive layer contains an acrylic copolymer obtained by copolymerizing a monomer mixture containing (a) a (meth)acrylic acid alkyl ester, (b) a hydroxyl group-containing monomer, and (c) a diketone group-containing monomer.

In one embodiment of the present invention, the pressure-sensitive adhesive layer contains an acrylic copolymer obtained by copolymerizing a monomer mixture containing 7.5 wt% to 20 wt% of the hydroxyl group-containing monomer (b).

In another embodiment of the present invention, the pressure-sensitive adhesive layer contains an acrylic copolymer obtained by copolymerizing a monomer mixture containing 2.5 wt% to 7.5 wt% of the diketone group-containing monomer (c).

In still another embodiment of the present invention, the pressure-sensitive adhesive layer further contains a plasticizer having compatibility with the acrylic copolymer and a weight ratio between the acrylic copolymer and the plasticizer is 1:0.1 to 1:2.

In still another embodiment of the present invention, the hydroxyl group-containing monomer (b) includes at least one selected from the group consisting of an N-hydroxyalkyl (meth)acrylamide and a hydroxyalkyl (meth)acrylate.

In still another embodiment of the present invention, the N-hydroxyalkyl (meth) acrylamide includes at least one selected from the group consisting of an N-(2-hydroxyethyl)acrylamide and an N-(2-hydroxyethyl)methacrylamide.

In still another embodiment of the present invention, the hydroxyalkyl (meth)acrylate includes a glycerin monomethacrylate.

In still another embodiment of the present invention, the diketone group-containingmonomer (c) includes at least one selected from the group consisting of an acetoacetyl group-containing (meth)acrylic monomer and a diacetone

acrylamide.

In still another embodiment of the present invention, the pressure-sensitive adhesive layer has a gel fraction of 25 wt% to 70 wt%.

In still another embodiment of the present invention, the acrylic copolymer self-cross-links to form a three-dimensional network structure without using any cross-linking agent.

According to another aspect of the present invention, a patch preparation is provided. The patch preparation includes the patch and a drug incorporated into the pressure-sensitive adhesive layer in the patch.

In one embodiment of the present invention, the drug includes a basic drug.

According to the present invention, the use of the acrylic copolymer obtained by copolymerizing the monomer mixture containing the (meth)acrylic acid alkyl ester, the hydroxyl group-containing monomer, and the diketone group-containing monomer in the pressure-sensitive adhesive layer can markedly improve the cohesive strength of the pressure-sensitive adhesive layer while maintaining its adhesion. As a result, there can be provided a patch showing sufficient pressure-sensitive adhesiveness upon attachment to a skin and capable of being released and removed from the skin after its use without causing stringing. Further, when a drug is incorporated into the pressure-sensitive adhesive layer, the above-mentioned acrylic copolymer and the drug do not cause any undesired reaction because the acrylic copolymer is substantially free of any acidic group. Therefore, when the patch is turned into a patch preparation, a patch preparation having excellent discharge property of a drug (especially a basic drug) can be obtained.

## DETAILED DESCRIPTION OF THE PREFERRED EMBODIMENTS

The patch of the present invention includes: a support; and a pressure-sensitive adhesive layer provided on at least one surface of the support. The pressure-sensitive adhesive layer contains an acrylic copolymer obtained by copolymerizing a monomer mixture containing (a) a (meth)acrylic acid alkyl ester, (b) a hydroxyl group-containing monomer, and (c) a diketone group-containing monomer. Hereinafter, each of the constituent components, materials, and the like are described in detail.

### A. Acrylic copolymer

### A-1. (Meth)acrylic acid alkyl ester

The above-mentioned (meth) acrylic acid alkyl ester (monomer (a)) is typically represented by the following formula (I).

[Chem. 1]

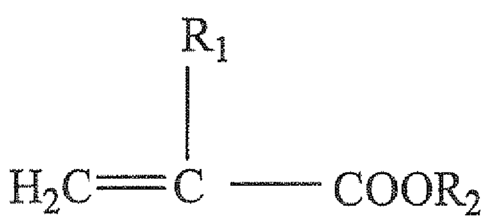

(I)

In the formula, $R_1$ represents a hydrogen atom or a methyl group, and $R_2$ represents an alkyl group. The alkyl group is preferably an alkyl group having 4 to 18 carbon atoms. When the alkyl group has 4 to 18 carbon atoms, a pressure-sensitive adhesive having a sufficiently low glass transition temperature is easily obtained. As a result, a patch having good adhesiveness (tack) is easily obtained.

Examples of the above-mentioned (meth)acrylic acid alkyl esters include those each having: a straight-chain alkyl group such as n-butyl, n-pentyl, n-hexyl, n-heptyl, n-octyl, n-nonyl, n-decyl, n-undecyl, n-dodecyl, or n-tridecyl; a branched-chain alkyl group such as isobutyl, isopentyl, isohexyl, isooctyl, or 2-ethylhexyl; or a cyclic alkyl group such as cyclopentyl, cyclohexyl, or cycloheptyl. They may be used alone or in combination.

Of the above-mentioned esters, a (meth) acrylic acid alkyl ester in which the alkyl group represented by $R_2$ in the formula (I) has 4 to 12 carbon atoms is more preferred. This is because of the following reason. In such a (meth) acrylic acid alkyl ester, a glass transition temperature can be successfully reduced, and as a result, good pressure-sensitive adhesiveness may be imparted to the pressure-sensitive adhesive layer to be obtained at normal temperature. To be specific, butyl acrylate, butyl methacrylate, 2-ethylhexyl acrylate, 2-ethylhexyl methacrylate, cyclohexyl acrylate, cyclohexyl methacrylate, or the like is preferred, and 2-ethylhexyl acrylate is most preferred. This is because of the

following reasons. That is, a polymer having a sufficiently low glass transition temperature (-70˚C) is obtained when the ester is polymerized. In addition, the ester is easily available.

The above-mentioned monomer (a) is preferably selected so that a homopolymer formed of the monomers may have a glass transition temperature of preferably -80˚C to -40˚C and particularly preferably -70˚C to -50˚C.

The content of the monomer (a) in the above-mentioned monomer mixture is preferably 50 wt% or more with respect to the total amount of the monomer mixture. When the content of the monomer (a) is 50 wt% or more, adhesiveness (tack) upon use of the mixture as a pressure-sensitive adhesive is good. The content of the monomer (a) is more preferably 60 wt% or more, particularly preferably 70 wt% or more. When the content of the monomer (a) is 60 wt% or more, better tack can be obtained. On the other hand, the content of the monomer (a) is preferably 90 wt% or less, more preferably 85 wt% or less with respect to the total amount of the monomer mixture. When the content of the monomer (a) in the monomer mixture is excessively large, there is a tendency that the physical properties of the copolymer to be obtained are close to the physical properties of a homopolymer of the above-mentioned monomer (a) and hence physical properties proper for the pressure-sensitive adhesive are hardly obtained.

A-2. Hydroxyl group-containing monomer

The above-mentioned hydroxyl group-containing monomer (monomer (b)) is representatively an N-hydroxyalkyl (meth)acrylamide represented by the following formula (II).

[Chem. 2]

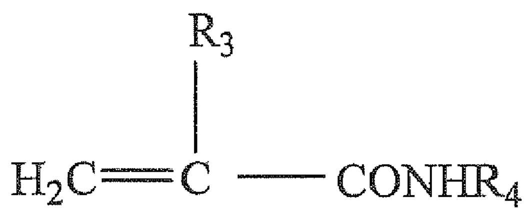
(II)

In the formula, $R_3$ represents a hydrogen atom or a methyl group, and $R_4$ represents a hydroxyalkyl group.

In the formula (II), the above-mentioned hydroxyalkyl group is preferably a hydroxyalkyl group having 2 to 4 carbon atoms. The alkyl group in the above-mentioned hydroxyalkyl group may be linear or branched. Examples of the N-hydroxyalkyl (meth)acrylamide represented by the formula (II) include N-(2-hydroxyethyl)acrylamide, N-(2-hydroxyethyl)methacrylamide, N-(2-hydroxypropyl)acrylamide, N-(2-hydroxypropyl)methacrylamide, N-(1-hydroxypropyl)acrylamide, N-(1-hydroxypropyl)methacrylamide, N-(3-hydroxypropyl)acrylamide, N-(3-hydroxypropyl)methacrylamide, N-(2-hydroxybutyl)acrylamide, N-(2-hydroxybutyl)methacrylamide, N-(3-hydroxybutyl)acrylamide, N-(3-hydroxybutyl)methacrylamide, N-(4-hydroxybutyl)acrylamide, and N-(4-hydroxybutyl)methacrylamide. They may be used alone or in combination. Preferred examples of the monomer (b) in the present invention include N-(2-hydroxyethyl)acrylamide and N-(2-hydroxyethyl)methacrylamide. Particularly preferred examples of the monomer (b) include N-(2-hydroxyethyl)acrylamide (HEAA). This is because HEAA has hydrophilicity and hydrophobicity in a well-balanced manner and allows the formation of a pressure-sensitive adhesive layer having an excellent balance in pressure-sensitive adhesiveness. For example, HEAA accounts for preferably 50 wt% or more, more preferably 70 wt% or more, still more preferably substantially all (i.e., 99.9 wt% or more) of the monomer (b).

Another representative example of the monomer (b) is a hydroxyalkyl (meth)acrylate. Specific examples thereof include 2-hydroxyethyl (meth)acrylate, 2-hydroxypropyl (meth)acrylate, 6-hydroxyhexyl (meth)acrylate, 4-hydroxybutyl (meth)acrylate, propylene glycol mono(meth)acrylate, 1,6-hexanediol mono (meth) acrylate, and glycerin mono (meth) acrylate. They may be used alone or in combination. In addition, the N-hydroxyalkyl (meth)acrylamide and the hydroxyalkyl (meth)acrylate may be used in combination.

The above-mentioned monomer (b) can contribute to an improvement in the cohesive strength of the pressure-sensitive adhesive by virtue of an interaction between its molecules. The content of the monomer (b) in the above-mentioned monomer mixture is preferably 7.5 wt% to 20 wt%, more preferably 7.5 wt% to 12 wt% with respect to the total weight of the monomer mixture. As long as the content of the monomer (b) falls within such range, a patch showing an additionally good cohesive strength and an additionally good adhesive strength is obtained. When the content of the monomer (b) is less than 7.5 wt%, an interaction between the hydroxyl group of the monomer (b) and the diketone group of the monomer (c) may be insufficient, and as a result, the cohesive strength of the pressure-sensitive adhesive layer to be obtained may be insufficient. When the content of the monomer (b) exceeds 20 wt%, the monomer mixture cannot be copolymerized in some cases.

A-3. Diketone group-containing monomer

Representative examples of the above-mentioned diketone group-containing monomer (monomer (c)) include an acetoacetyl group-containing (meth) acrylic monomer and diacetone acrylamide. Specific examples of the acetoacetyl group-containing (meth) acrylic monomer include acetoacetoxyethyl methacrylate, acetoacetoxyethyl acrylate, acetoacetoxypropyl methacrylate, acetoacetoxypropyl acrylate, acetoacetoxybutyl methacrylate, and acetoacetoxybutyl acrylate. They may be used alone or in combination.

It is estimated that the above-mentioned monomer (c) causes the acrylic copolymer to be obtained to self-cross-link by virtue of an interaction between its diketone group and the hydroxyl group of the above-mentioned monomer (b) so that a three-dimensional network structure may be formed in the pressure-sensitive adhesive layer. Therefore, the cohesive strength of the pressure-sensitive adhesive can be markedly improved by copolymerizing the monomer (b) and the monomer (c). As a result, stringing upon release and removal of the patch from a skin after its use can be significantly prevented. On the other hand, such excellent effect is not obtained even by blending a copolymer obtained from a monomer mixture containing the diketone group-containing monomer and a copolymer obtained from a monomer mixture containing the hydroxyl group-containing monomer. It is estimated from the foregoing that a completely different structure is formed in the pressure-sensitive adhesive layer in the case of the blend. As described above, such effect that the cohesive strength of the pressure-sensitive adhesive is markedly improved by copolymerizing the diketone group-containing monomer and the hydroxyl group-containing monomer is knowledge which was not obtained until the copolymer obtained by such copolymerization was applied to the pressure-sensitive adhesive layer of the patch, and is hence an unexpected excellent effect.

The content of the monomer (c) in the above-mentioned monomer mixture is preferably 2.5 wt% to 7.5 wt%, more preferably 4 wt% to 6 wt% with respect to the total weight of the monomer mixture. When the content of the monomer (c) is less than 2.5 wt%, an interaction between the diketone group of the monomer (c) and the hydroxyl group of the monomer (b) may be insufficient, and as a result, the cohesive strength of the pressure-sensitive adhesive layer to be obtained may be insufficient. When the content of the monomer (c) exceeds 7.5 wt%, the adhesiveness of the pressure-sensitive adhesive layer to be obtained may be insufficient.

The total content of the monomers (a) to (c) in the above-mentioned monomer mixture is preferably about 90 wt% or more, more preferably 95 wt% or more, still more preferably 98 wt% or more, particularly preferably substantially 100 wt% (that is, a copolymer obtained by copolymerizing only the monomers (a) to (c)) with respect to the total weight of the monomer mixture. The use of a pressure-sensitive adhesive obtained from a copolymer of such composition can provide a patch showing a good cohesive strength and a good adhesive strength upon bonding to a skin surface.

A-4. Any other monomer

The above-mentioned monomer mixture may contain a vinyl-based monomer copolymerizable with the monomers (a) to (c) as well as these monomers. The addition of a proper vinyl-based monomer enables the adjustment of the adhesion and cohesive strength of each of a patch and a patch preparation, and the adjustment of the solubility and discharge property of a drug.

When the vinyl-basedmonomer is used, its content in the monomer mixture is preferably 10 wt% or less, more preferably 5 wt% or less with respect to the total amount of the monomer mixture. When the content of the vinyl-based monomer exceeds 10 wt%, the tack or adhesion of each of the patch and the patch preparation to be obtained may reduce.

Any one of, for example, the vinyl ethers such as methyl vinyl ether and ethyl vinyl ether, and the vinyl-based monomers each having a heterocycle containing a nitrogen atom such as N-vinyl-2-pyrrolidone, 1-vinyl caprolactam, 2-vinyl-2-piperidone, and 1-vinylimidazole can be used as the vinyl-based monomer. They may be used alone or in combination. Of the vinyl-based monomers, a vinyl-based monomer having a heterocycle containing a nitrogen atom is preferably used.

In the present invention, the above-mentioned monomer mixture is preferably substantially free of any carboxyl group-containing monomer. The carboxyl group-containing monomer is representatively, for example, an ethylenically unsaturated monomer (representatively a vinyl-based monomer) having at least one carboxyl group (which may be of such a form that an anhydride is formed) in a molecule thereof. Specific examples of the carboxyl group-containing monomer include: ethylenically unsaturated monocarboxylic acids such as (meth)acrylic acid and crotonic acid; ethylenically unsaturated dicarboxylic acids such as maleic acid, itaconic acid, and citraconic acid; and anhydrides of ethylenically unsaturated dicarboxylic acids such as maleic anhydride and itaconic anhydride. It should be noted that the expression "the monomer mixture is substantially free of any monomer having a carboxyl group" in the specification comprehends not only the case where the monomer mixture is completely free of any monomer having a carboxyl group but also the case where the content of such monomer is 0.1 wt% or less with respect to the total amount of the monomer mixture.

Further, in the present invention, theabove-mentionedmonomer mixture preferably not only is substantially free

of any carboxyl group-containing monomer but also is substantially free of any monomer having an acidic group except a carboxyl group (such as a sulfo group or a phosphate group). That is, it is preferred that the above-mentioned monomer mixture be completely free of any carboxyl group-containing monomer and any monomer having any other acidic group or contain these monomers at a content of 0.1 wt% or less with respect to the total amount of the monomer mixture. The incorporation of a medical active component such as a drug into the pressure-sensitive adhesive layer containing the copolymer obtained by copolymerizing such monomer mixture can forestall, for example, the denaturation of the active component and the inhibition of its movement in the pressure-sensitive adhesive layer due to a reaction with a carboxyl group or the like.

A-5. Method of polymerizing acrylic copolymer

A polymerization method for obtaining the acrylic copolymer from the above-mentioned monomer mixture is not particularly limited, and any appropriate polymerization method can be adopted. For example, a polymerization method involving using a thermal polymerization initiator (a thermal polymerization method such as a solution polymerization method, an emulsion polymerization method, or a bulk polymerization method), or a polymerization method involving applying an active energy ray (also referred to as "high-energy ray") such as light or radiation can be adopted.

Of the above-mentioned polymerization methods, the solution polymerization method can be preferably adopted because the method is excellent in, for example, workability and quality stability. The mode of the solution polymerization is not particularly limited, and any appropriate mode can be adopted. Specifically, any appropriate monomer supply method, any appropriate polymerization condition (such as a polymerization temperature, a polymerization time, or a polymerization pressure), and any appropriate material to be used (such as a polymerization initiator or a surfactant) can be adopted. Any one of, for example, a collective loading system involving supplying the total amount of the monomer mixture to a reaction vessel in one stroke, a continuous supply (dropping) system, and a split supply (dropping) system can be adopted as the above-mentioned monomer supply method. A preferred mode is, for example, such a mode that a solution prepared by dissolving the total amount of the monomer mixture and an initiator in a solvent is supplied to a reaction vessel and then the monomer mixture is collectively polymerized (collective polymerization). Such collective polymerization is preferred because a polymerization operation and process control are easy. Another preferred mode is, for example, such a mode that an initiator (typically a solution prepared by dissolving the initiator in a solvent) is prepared in a reaction vessel and then a solution prepared by dissolving the monomer mixture in a solvent is polymerized while being dropped into the reaction vessel (dropping polymerization or continuous polymerization) . Part of themonomermixture (part of the components and/or part of the amount) may be loaded into a reaction vessel typically together with a solvent, and the remaining monomer mixture may be dropped into the reaction vessel. When the monomer mixture containing the monomer (b) at a content of 15 wt% or more is polymerized, the dropping polymerization is more preferably employed from the viewpoint of the ease with which a polymerization reaction is uniformly advanced.

Examples of the thermal polymerization initiator include: azo-based compounds (azo-based initiators) such as 2,2'-azobisisobutyronitrile, 2,2'-azobis-2-methylbutyronitrile, dimethyl 2,2'-azobis(2-methylpropionate), 4,4'-azobis-4-cyanovaleric acid, azobisisovaleronitrile, 2,2'-azobis(2-amidinopropane) dihydrochloride, 2,2'-azobis[2-(5-methyl-2-imidazoiin-2-yl)propane] dihydrochloride, 2,2'-azobis(2-methylpropionamidine) disulfate, 2,2'-azobis(N,N'-dimethyleneisobutylamidine) dihydrochloride, and 2,2'-azobis[N-(2-carboxyethyl)-2-methylpropionamidine] hydrate; persulfates such as potassium persulfate and ammonium persulfate; peroxides (peroxide-based initiators) such as dibenzoyl peroxide, tert-butyl permaleate, t-butyl hydroperoxide, and hydrogen peroxide; substituted ethane-based initiators such as phenyl-substituted ethane; and redox-based initiators such as a mixture of a persulfate and sodium hydrogen sulfite and a mixture of a peroxide and sodium ascorbate. When the monomer mixture is polymerized by the thermal polymerization method, the polymerization temperature is preferably about 20°C to about 100°C, more preferably about 40°C to about 80°C.

The polymerization method involving applying light (typically ultraviolet light) is typically per formed with a photopolymerization initiator. The photopolymerization initiator is not particularly limited, and there may be used, for example, a ketal-based photopolymerization initiator, an acetophenone-based photopolymerization initiator, a benzoin ether-based photopolymerization initiator, an acylphosphine oxide-based photopolymerization initiator, an $\alpha$-ketol-based photopolymerization initiator, an aromatic sulfonyl chloride-based photopolymerization initiator, a photoactive oxime-based photopolymerization initiator, a benzoin-based photopolymerization initiator, a benzyl-based photopolymerization initiator, a benzophenone-based photopolymerization initiator, and a thioxanthone-based photopolymerization initiator. Those photopolymerization initiators may be used alone or in combination.

Examples of the ketal-based photopolymerization initiator include 2,2-dimethoxy-1,2-diphenylethan-1-one [such as one under the trade name "Irgacure 651" (manufactured by Ciba Japan KK)]. Examples of the acetophenone-based photopolymerization initiator include 1-hydroxycyclohexyl phenyl ketone [such as one under the trade name "Irgacure 184" (manufactured by Ciba Japan KK)], 2,2-diethoxyacetophenone, 2,2-dimethoxy-2-phenylacetophenone, 4-phenoxydichloroacetophenone, and 4-(t-butyl) dichloroacetophenone. Examples of the benzoin ether-based photopolymerization initiator include benzoin methyl ether, benzoin ethyl ether, benzoin propyl ether, benzoin isopropyl ether, and benzoin isobutyl ether. Examples of the acylphosphine oxyde-based photopolymerization initiator include one under the trade name "Lucirin TPO" (manufactured by BASF). Examples of the α-ketol-based photopolymerization initiator include 2-methyl-2-hydroxypropiophenone and 1-[4-(2-hydroxyethyl)phenyl]-2- methylpropan-1-one. Examples of the aromatic sulfonyl chloride-based photopolymerization initiator include 2-naphthalenesulfonyl chloride. Examples of the optically active oxime-based photopolymerization initiator include 1-phenyl-1,1-propanedione-2-(o-ethoxycarbonyl)-oxime. Examples of the benzoin-based photopolymerization initiator include benzoin. Examples of the benzyl-based photopolymerization initiator include benzyl. Examples of the benzophenone-based photopolymerization initiator include benzophenone, benzoylbenzoic acid, 3,3'-dimethyl-4-methoxybenzophenone, polyvinylbenzophenone, and α-hydroxycyclohexyl phenyl ketone. Examples of the thioxanthone-based photopolymerization initiator include thioxanthone, 2-chlorothioxanthone, 2-methylthioxanthone, 2,4-dimethylthioxanthone, isopropylthioxanthone, 2,4-diisopropylthioxanthone, and dodecylthioxanthone.

The usage of the above-mentioned polymerization initiator is not particularly limited. For example, the usage of the polymerization initiator is preferably about 0.01 part by weight to about 2 parts by weight, more preferably about 0.01 part by weight to about 1 part by weight with respect to 100 parts by weight of the total amount of the monomer mixture.

B. Pressure-sensitive adhesive layer

The above-mentioned pressure-sensitive adhesive layer is formed of a composition containing the acrylic copolymer described in the above-mentioned section A (hereinafter referred to as "pressure-sensitive adhesive layer-forming composition"). The thickness of the pressure-sensitive adhesive layer is preferably 10 μm to 400 μm, more preferably 20 μm to 200 μm, still more preferably 30 μm to 100 μm. The pressure-sensitive adhesive layer may be continuously formed, or may be formed so as to be of a predetermined pattern (for example, a regular pattern such as a dot- or stripe-shaped pattern, or a random pattern) depending on purposes.

The pressure-sensitive adhesive layer (substantially the pressure-sensitive adhesive layer-forming composition) can preferably further contain a plasticizer having compatibility with the above-mentioned acrylic copolymer. The plasticizer can plasticize the pressure-sensitive adhesive layer to provide a feeling of softness. As a result, when the pressure-sensitive adhesive containing the above-mentioned acrylic copolymer is used as a pressure-sensitive adhesive layer, pain or skin irritation resulting from a skin adhesive strength can be reduced upon release of a patch or patch preparation such as a pressure-sensitive adhesive tape or a transdermally absorbable preparation from a skin. Therefore, any component can be used as the plasticizer without any particular limitation as long as the component has a plasticizing action. It should be noted that a component having an absorption-promoting action is preferably used for improving transdermal absorption property when a drug is incorporated into the pressure-sensitive adhesive layer.

Examples of the above-mentioned plasticizer include: plant fats and oils such as olive oil, castor oil, and palm oil; animal fats and oils such as lanolin; organic solvents such as dimethyl decyl sulfoxide, methyl octyl sulfoxide, dimethyl sulfoxide, dimethylformamide, dimethylacetamide, dimethyllaurylamide, methylpyrrolidone, and dodecylpyrrolidone; liquid surfactants such as a polyoxyethylene sorbitan fatty acid ester, a sorbitan fatty acid ester, and a polyoxyethylene fatty acid ester; plasticizers such as diisopropyl adipate, a phthalate, and diethyl sebacate; hydrocarbons such as squalane and liquid paraffin; fatty acid alkyl esters such as ethyl oleate, isopropyl palmitate, octyl palmitate, isopropyl myristate, isotridecyl myristate, and ethyl laurate, preferably esters of a fatty acid having 8 to 18 (more preferably 12 to 16) carbon atoms and a monohydric alcohol having 1 to 18 carbon atoms; fatty acid esters of polyhydric alcohols such as a glycerin fatty acid ester and a propylene glycol fatty acid ester; ethoxylated stearyl alcohol; and a pyrrolidone carboxylic acid fatty acid ester. They may be used alone or in combination.

The above-mentioned plasticizer can be incorporated into the pressure-sensitive adhesive layer at a weight ratio of preferably 1:0.1 to 1:2, more preferably 1:0.1 to 1:0.7 with respect to the acrylic copolymer. As long as the amount of the plasticizer falls within such range, a pressure-sensitive adhesive layer showing a small stimulus to a skin can be obtained. It should be noted that the plasticizer is preferably incorporated in as large an amount as possible to such an extent that the adhesiveness of the pressure-sensitive adhesive layer is not impaired.

In addition, the pressure-sensitive adhesive layer (substantially the pressure-sensitive adhesive layer-forming composition) may further contain any other components as far as the effect of the present invention is not impaired. Examples of such arbitrary components include antioxidants such as ascorbic acid, tocopherol acetate, natural vitamin E, dibutylhydroxytoluene, and butylhydroxyanisole; amine-ketone-based anti-aging agents such as 2,6-tert-butyl-4-methylphenol; aromatic secondary amine-based anti-aging agents such as N,N'-di-2-naphthyl-p-phenylenediamine; monophenol-based anti-aging agents such as a 2,2,4-trimethyl-1,2-dihydroquinoline polymer; bisphenol-based anti-aging agents such as 2,2'-methylenebis(4-ethyl-6-tert-butylphenol); polyphenol-based anti-aging agents such as 2, 5-tert-butylhydroquinone; fillers such as kaolin, hydrous silicon dioxide, zinc oxide, and starch acrylate 1000; softening agents such as propylene glycol, polybutene, and macrogol 1500; antiseptics such as benzoic acid, sodium benzoate, chlorhexidine hydrochloride, sorbic acid, methyl paraoxybenzoate, and butyl paraoxybenzoate; coloring agents such as

yellow iron oxide, yellow iron(III) oxide, iron(III) oxide, black iron oxide, carbon black, carmine, β-carotene, copper chlorophyll, Food Blue No.1, Food Yellow No.4, Food Red No. 2, and licorice extract; cooling agents such as fennel oil, d-camphor, dl-camphor, mint oil, d-borneol, and l-menthol; and perfumes such as spearmint oil, clove oil, vanillin, bergamot oil, and lavender oil. The kind and amount of other components to be incorporated may be appropriately set depending on purposes.

The pressure-sensitive adhesive layer has a gel fraction of preferably 25 wt% to 70 wt%, more preferably 30 wt% to 60 wt%, still more preferably 30 wt% to 50 wt%. As long as the gel fraction falls within such range, a sufficient cohesive strength is imparted to the pressure-sensitive adhesive layer, and there is no possibility that an adhesive residue, stringing, a strong skin stimulus, or the like resulting from a cohesive failure arises at the time of the release of the patch. The incorporation of the above-mentioned copolymer and the above-mentioned plasticizer into the pressure-sensitive adhesive layer can achieve the above-mentioned gel fraction.

The term "gel fraction" refers to a ratio of the weight of insoluble matter obtained when the pressure-sensitive adhesive layer is immersed in an organic solvent such as ethyl acetate to the total weight of components involved in the cross-linking of the pressure-sensitive adhesive layer. The gel fraction can be determined from the weight of the insoluble matter obtained by immersing the pressure-sensitive adhesive layer in the organic solvent such as ethyl acetate at normal temperature (23°C) for a predetermined time period by using the following equation:

$$\text{Gel fraction (wt\%)} = (W_2 \times 100) / (W_1 \times A/B)$$

where A represents the weight of the polymer and the cross-linking agent, B represents the total weight of the constituent components of the pressure-sensitive adhesive layer, $W_1$ represents the weight of the pressure-sensitive adhesive layer as a sample, and $W_2$ represents the weight of the insoluble matter after the immersion of the pressure-sensitive adhesive layer as a sample in the organic solvent.

In the present invention, the pressure-sensitive adhesive layer (substantially the pressure-sensitive adhesive layer-forming composition) may be subjected to a physical cross-linking treatment based on, for example, the application of radiation such as the application of UV light or the application of an electron beam, or to a chemical cross-linking treatment with any one of various cross-linking agents as required. Any appropriate cross-linking agent can be selected as one of the above-mentioned cross-linking agents. For example, an isocyanate-based compound (isocyanate-based cross-linking agent), an epoxy-based cross-linking agent, an aziridine-based cross-linking agent, a melamine-based cross-linking agent, a peroxide-based cross-linking agent, an oxazoline-based cross-linking agent, a urea-based cross-linking agent, an amino-based cross-linking agent, a carbodiimide-based cross-linking agent, or a coupling agent-based cross-linking agent (such as a silane coupling agent) can be used. They may be used alone or in combination. The use of any such cross-linking agent to cross-link (cure) the pressure-sensitive adhesive layer enables the adjustment of the gel fraction of the pressure-sensitive adhesive layer. In the present invention, the cross-linking agent to be used for cross-linking the pressure-sensitive adhesive layer is added in an amount of preferably about 0.01 part by weight to about 5 parts by weight, more preferably about 0.01 part by weight to about 2 parts by weight with respect to 100 parts by weight of the acrylic copolymer. It should be noted that in the present invention, the use of the acrylic copolymer described in the above-mentioned section A in the pressure-sensitive adhesive layer can provide a desired gel fraction while obviating the need for the use of any cross-linking agent.

C. Support

The above-mentioned support is not particularly limited. The support is preferably such a support that a reduction in content of a component (including an active component such as a drug, or an additive) incorporated into the pressure-sensitive adhesive layer does not occur owing to the loss of the component from the back surface of the support as a result of its permeation through the support, that is, a support which is formed of a material impermeable to the component incorporated into the pressure-sensitive adhesive layer.

Examples of the support which may be used in the patch and patch preparation of the present invention include: polyester resins such as polyethylene terephthalate; polyamide-based resins such as nylon; olefin-based resins such as Saran (registered trade mark), polyethylene, polypropylene, and Surlyn (registered trade mark); vinyl-based resins such as an ethylene-vinyl acetate copolymer, polyvinyl chloride, and polyvinylidene chloride; acrylic resins such as an ethylene-ethyl acrylate copolymer; fluorinated carbon resins such as polytertrafluoroethylene; single films of metallic foil and the like, and laminated films thereof. The support has a thickness of preferably 10 μm to 500 μm, more preferably 10 μm to 200 μm.

The above-mentioned support is preferably a laminated sheet of a nonporous sheet formed of any one of the above-mentionedmaterials and a porous sheet. Such constitution can improve adhesiveness (anchoring property) between the support and the pressure-sensitive adhesive layer. In this case, the pressure-sensitive adhesive layer is preferably formed on the side of the porous sheet. The above-mentioned porous sheet is not particularly limited as long as the sheet can improve anchoring property between the support and the pressure-sensitive adhesive layer. Examples of the porous sheet include paper, a woven fabric, a nonwoven fabric, and a mechanically perforated sheet. Of those, paper, the woven fabric, or the nonwoven fabric is particularly preferred. The porous sheet preferably has a thickness of 10 μm to 500 μm. With such thickness, the anchoring property is improved, and the flexibility of the pressure-sensitive adhesive layer is excellent. In addition, when the woven fabric or the nonwoven fabric is used as the porous sheet, the porous sheet has a mass per unit area of preferably 5 $g/m^2$ to 30 $g/m^2$, more preferably 8 $g/m^2$ to 20 $g/m^2$. This is because the anchoring property is improved. It should be noted that when the support is the above-mentioned laminated sheet, the thickness of the nonporous sheet is preferably 1 μm to 25 μm.

Of the above-mentioned supports, a particularly suitable support is a laminated film of a polyester film (preferably a polyethylene terephthalate film) having a thickness of 1.5 μm to 6 μm and a nonwoven fabric made of a polyester (preferably a polyethylene terephthalate) having a mass per unit area of 8 $g/m^2$ to 20 $g/m^2$.

D. Patch and patch preparation

A patch of the present invention is obtained by forming, on at least one surface of the support described in the above-mentioned section C, the pressure-sensitive adhesive layer from the pressure-sensitive adhesive described in each of the above-mentioned sections A and B. The patch of the present invention can be provided as, for example, a sheet-, film-, or pad-shaped, medical or sanitary patch, and can find use in applications such as the protection of a lesion site or wounded site of a skin including an alternative to a gauze in a bandage and an alternative to a nonwoven fabric in a wound-covering dressing.

A patch preparation of the present invention is obtained by incorporating a drug into the pressure-sensitive adhesive layer in the above-mentioned patch of the present invention. The patch preparation of the present invention is provided as a transdermally absorbable preparation, and is provided as, for example, a matrix-type patch preparation or a reservoir-type patch preparation, in particular, as the matrix-type patch preparation.

In the patch preparation of the present invention, a drug that is incorporated into the pressure-sensitive adhesive layer is representatively a drug that can be transdermally administered. The kind of the drug to be incorporated into the pressure-sensitive adhesive layer can be appropriately selected depending on purposes. Specific examples of the drug include a type of drug that can be transdermally administered, including a corticosteroid drug, a non-steroidal anti-inflammatory drug, an antirheumatic drug, a sleeping drug, an antipsychotic drug, an antidepressant, a mood stabilizer, apsychostimulant, anantianxietydrug, an antiepileptic drug, a migraine therapeutic drug, a Parkinson's disease therapeutic drug, a cerebral circulation/metabolism improver, an anti-dementia drug, an autonomic drug, a muscle relaxant, a hypotensive drug, a diuretic drug, a hypoglycemic drug, a hyperlipidemia therapeutic drug, an arthrifuge, a general anesthetic, a local anesthetic, an antibacterial drug, an antifungal drug, an antiviral drug, an anti-parasite drug, a vitamin drug, an angina pectoris therapeutic drug, a vasodilator, an antiarrhythmic drug, an antihistaminic drug, a mediator release inhibitor, a leukotriene antagonist, a female hormone drug, a thyroid hormone drug, an antithyroid drug, an antiemetic, ananti-dizziness drug, a bronchodilator, an antitussive drug, an expectorant, and a smoking cessation adjunct. Of those, a drug whose discharge property extremely reduces in a pressure-sensitive adhesive layer containing a carboxyl group can be suitably incorporated into the patch preparation of the present invention in view of the characteristics of the copolymer in the pressure-sensitive adhesive layer.

In the present invention, it is advantageous to use a basic drug as the drug from such a viewpoint that a patch preparation having high skin permeability is obtained. The basic drug means a drug having a basic group in any one of its molecules. In the case of the patch preparation of the present invention containing the acrylic copolymer substantially free of a carboxyl group in the pressure-sensitive adhesive layer, for example, the inhibition of the movement of the basic drug in the pressure-sensitive adhesive layer caused by a reaction between the basic group of the basic drug and a carboxyl group can be suppressed. From such viewpoint, the basic drug is preferably a basic drug having a basic nitrogen atom, more preferably a drug having a primary, secondary, or tertiary amino group.

The content of the above-mentioned drug in the patch preparation of the present invention can be appropriately set depending on, for example, the kind of the drug and a purpose of its administration, and the age, sex, and symptom of a patient. The content of the drug in the pressure-sensitive adhesive layer is typically about 0.1 wt% to 40 wt%, preferably about 0.5 wt% to 30 wt%. In general, when the content is less than 0.1 wt%, the discharge of an amount of the drug effective for a treatment cannot be expected, and when the content exceeds 40 wt%, a therapeutic effect is not improved in most cases, and an economic disadvantage arises, though a preferred content cannot be uniquely defined because the content varies depending on the selected drug.

A method of producing each of the patch and patch preparation of the present invention is not particularly limited, and an approach conventionally employed in the field can be employed. Hereinafter, specific description is given by taking a matrix-type patch preparation as an embodiment mode of the patch preparation of the present invention as

an example. First, the acrylic copolymer, the plasticizer, the drug, and the like described above are dissolved or dispersed in a solvent. Next, a cross-linking agent is added to the above-mentioned solution or dispersion liquid as required. Thus, an application liquid contain ing a pressure-sensitive adhesive layer-forming composition is obtained. The pressure-sensitive adhesive layer is formed by applying the application liquid to at least one surface of the support and drying the application liquid. Further, a release liner to be described later can be laminated by crimping. Alternatively, the patch preparation can be produced by: applying the pressure-sensitive adhesive layer-forming composition onto the release liner; drying the applied composition to form the pressure-sensitive adhesive layer on the surface of the release liner; and attaching the support onto the pressure-sensitive adhesive layer by crimping.

Examples of the above-mentioned release liner include: glassine paper, polyethylene, polypropylene, polyester, polyethylene terephthalate, polystyrene, an aluminum film, a foamed polyethylene film, and a foamed polypropylene film; and a laminated product of two or more selected from them and products obtained by subjecting them to a silicone processing and an emboss processing. The release liner has a thickness of preferably 10 μm to 200 μm, more preferably 25 μm to 100 μm.

The above-mentioned release liner is preferably a release liner made of a polyester (especially polyethylene terephthalate) resin in terms of barrier property and a price. Further, in this case, its thickness is preferably about 25 μm to 100 μm in terms of handleability.

The application of the pressure-sensitive adhesive layer-forming composition can be performed with any conventionally used coater such as a gravure roll coater, a reverse roll coater, a kiss-roll coater, a dip roll coater, a bar coater, a knife coater, or a spray coater. The above-mentioned composition is preferably dried under heating from the viewpoints of, for example, the acceleration of a cross-linking reaction and an improvement in production efficiency. The drying temperature can vary depending on the kind of the support to which the pressure-sensitive adhesive layer-forming composition is applied. The drying temperature is, for example, about 40˚C to about 150˚C.

In addition, after the production of the patch or the patch preparation by such method as described above, aging may be performed at a temperature equal to or more than room temperature for the purposes of: completing a cross-linking reaction; and improving anchoring property between the pressure-sensitive adhesive layer and the support. An aging temperature is preferably 40˚C to 80˚C, more preferably 50˚C to 80˚C. An aging time is preferably 12 to 96 hours, more preferably 24 to 72 hours. When the aging time or temperature is smaller than the lower limit, the cross-linking reaction may be insufficient. When the aging time or temperature is larger than the upper limit, a component of the pressure-sensitive adhesive layer (such as the drug) may be adversely affected.

## EXAMPLES

Hereinafter, the present invention is described in more detail by way of examples. However, the present invention is not limited to those examples. It should be noted that, unless otherwise stated, the terms "part(s)" and "%" in the examples refer to "part(s) by weight" and "wt%," respectively.

### <Example 1>

85 Parts of 2-ethylhexyl acrylate (hereinafter referred to as "2-EHA") as the monomer (a), 10 parts of N-hydroxyethylacrylamide (hereinafter referred to as "HEAA") as the monomer (b), 5 parts of acetoacetoxyethyl methacrylate (hereinafter referred to as "AAEM") as the monomer (c), and 0.2 part by weight of 2,2'-azobisisobutyronitrile (AIBN) as a polymerization initiator were loaded into a separable flask provided with a reflux condenser, a nitrogen gas-introducing tube, a temperature gauge, a dropping funnel, and a stirring machine. 122 Parts of ethyl acetate were charged as a solvent into the flask, and then the contents were stirred at room temperature for 1 hour while nitrogen gas bubbling (50 mL/min) was performed. After that, the contents were heated, and were then subjected to a reaction in a stream of a nitrogen gas for 6 hours while such control that the temperature of the contents was kept at 60˚C was performed. After that, the contents were subjected to a reaction at 75 ˚C for an additional eighteen hours. A solution of an acrylic copolymer (2-EHA/HEAA/AAEM=85/10/5) was obtained by solution polymerization based on the above-mentioned system.

40 Parts of isopropyl myristate (IPM) were added as a plasticizer to 60 parts (solid content) of the acrylic copolymer obtained in the foregoing. Ethyl acetate was added to adjust the viscosity of the mixture. Thus, an application liquid containing a pressure-sensitive adhesive layer-forming composition was prepared. The composition was applied to the release surface of a release liner made of a polyethylene terephthalate (PET) film having a thickness of 75 μm with an applicator so as to have a thickness after its drying of 60 μm, and was then dried at 100 ˚C for 3 minutes. Thus, a pressure-sensitive adhesive layer was formed. A laminate of a PET film having a thickness of 2 μm and a PET nonwoven fabric having a mass per unit area of 14 $g/m^2$ was used as the support. The nonwoven fabric surface of the support was attached to the pressure-sensitive adhesive layer by crimping, and then the resultant was sealed with an aluminum packaging material and subjected to aging at 60˚C for 48 hours. Thus, a patch was obtained.

<Example 2>

A patch was obtained in the same manner as in Example 1 except that acetoacetoxybutyl acrylate (hereinafter referred to as "AABUA") was used instead of AAEM.

<Example 3>

A patch was obtained in the same manner as in Example 1 except that glycerin monomethacrylate (hereinafter referred to as "GLM") was used instead of HEAA.

<Example 4>

A patch was obtained in the same manner as in Example 1 except that: an acrylic copolymer of the following formulation was used; and the usage of IPM was changed to 20 parts.
2-EHA: 90 parts, HEAA: 5 parts, AAEM: 5 parts

<Example 5>

A patch was obtained in the same manner as in Example 1 except that an acrylic copolymer of the following formulation was used.
2-EHA: 80 parts, HEAA: 10 parts, AAEM: 10 parts

<Comparative Example 1>

A patch was obtained in the same manner as in Example 1 except that: an acrylic copolymer of the following formulation was used; and the usage of IPM was changed to 20 parts.
2-EHA: 95 parts, HEAA: 5 parts

<Comparative Example 2>

A patch was obtained in the same manner as in Example 1 except that: an acrylic copolymer of the following formulation was used; and the usage of IPM was changed to 20 parts.
2-EHA: 95 parts, AAEM: 5 parts

<Comparative Example 3>

A patch was obtained in the same manner as in Example 1 except that an acrylic copolymer of the following formulation was used.
2-EHA: 70 parts, 1-vinyl-2-pyrrolidone (hereinafter referred to as "VP"): 20 parts, HEAA: 10 parts

<Comparative Example 4>

A patch was obtained in the same manner as in Example 1 except that an acrylic copolymer of the following formulation was used.
2-EHA: 75 parts, VP: 20 parts, AAEM: 5 parts

<Comparative Example 5>

A patch was obtained as described below. An acrylic copolymer A (2-EHA/HEAA=95/5) was obtained in the same manner as in Comparative Example 1. Separately, an acrylic copolymer B (2-EHA/AAEM=95/5) was obtained in the same manner as in Comparative Example 2. The copolymer A and the copolymer B were blended at a solid content weight ratio A/B of 75/25, and then 20 parts of IPM were added to 80 parts (solid content) of the blend. The subsequent procedure was the same as that of Example 1.

<Comparative Example 6>

Apatch was obtained in the same manner as in Comparative Example 5 except that the blending ratio A/B of the copolymer A to the copolymer B was changed to 50/50 (solid content weight ratio).

<Comparative Example 7>

A patch was obtained in the same manner as in Comparative Example 5 except that the blending ratio A/B of the copolymer A to the copolymer B was changed to 25/75 (solid content weight ratio).

<Evaluation>

(1) Gel fraction

The weight ($W_1$) (g) of the pressure-sensitive adhesive layer of a patch sample having an area of 10 $cm^2$ was measured. Next, the sample was immersed in 100 ml of ethyl acetate for 24 hours so that solvent soluble matter was extracted. After that, the sample was taken out and dried. The weight ($W_2$) (g) of the pressure-sensitive adhesive layer after the drying was measured, and then a gel fraction was calculated from the following equation.

$$\text{Gel fraction (wt\%)} = (W_2 \times 100) / (W_1 \times A/B)$$

where A represented the weight of the polymer and the cross-linking agent, B represented the total weight of the constituent components of the pressure-sensitive adhesive layer, $W_1$ represented the weight of the pressure-sensitive adhesive layer as a sample, and $W_2$ represented the weight of the insoluble matter after the immersion of the pressure-sensitive adhesive layer as a sample in the organic solvent.

(2) Finger tack (stringing) test

The release liner was released from each of the patches obtained in the examples and the comparative examples, and was then mounted on a horizontal table so that the surface of a plaster (pressure-sensitive adhesive layer) was a top surface. A finger was pressed against the plaster, and then a tacky feeling and the degree of stringing were evaluated. Evaluation criteria are as described below.
○ : The plaster has a cohesive strength, a sufficient tacky feeling is obtained, and no stringing is observed.
Δ : The plaster has a cohesive strength and no stringing of the plaster is observed, but the tacky feeling is somewhat insufficient. ×: The plaster has no cohesive strength and the stringing of the plaster is observed.

(3) Skin attachment test

Each of the patches obtained in the examples and the comparative examples was punched into a sample having an area of 10 $cm^2$. The sample was attached to the chest of a participant in the experiment, and then its adhesiveness for 24 hours was observed. Evaluation criteria are as described below.
○ : None of floating and an adhesive residue was observed.
Δ : No adhesive residue was observed but the floating of an end occurred.
×: An adhesive residue was observed.

Table 1 shows the results of the evaluations of the above-mentioned items (1) to (3).

[Table 1]

| | Gel fraction (%) | Stringing | Skin attachment property |
|---|---|---|---|
| Example 1 | 38.4 | ○ | ○ |
| Example 2 | 36.6 | ○ | ○ |
| Example 3 | 33.3 | ○ | ○ |
| Example 4 | - | Δ | Δ |
| Example 5 | 45.0 | Δ | Δ |
| Comparative Example 1 | 0.8 | × | × |
| Comparative Example 2 | 1.2 | × | × |
| Comparative Example 3 | 1.3 | × | × |
| Comparative Example 4 | 3.7 | × | × |

(continued)

| | Gel fraction (%) | Stringing | Skin attachment property |
|---|---|---|---|
| Comparative Example 5 | 6.1 | × | × |
| Comparative Example 6 | 1.7 | × | × |
| Comparative Example 7 | 0.5 | × | × |
| * The gel fraction of Example 4 is not measured. | | | |

As is apparent from Table 1, the pressure-sensitive adhesive layer of each of the patches of the examples of the present invention has a high gel fraction, and the attachment drug is excellent in each of stringiness, skin attachment property, and an adhesive strength. As the pressure-sensitive adhesive layer of each of the patches of the examples of the present invention has a high gel fraction even when no cross-linking agent is used, it is estimated that the use of a hydroxyl group-containing monomer and a diketone group-containing monomer as copolymer components causes the acrylic copolymer that constitutes the pressure-sensitive adhesive layer to self-cross-link to form a three-dimensional network structure. Further, as is apparent from comparison between any one of Examples 1 to 3 and each of Examples 4 and 5, the optimization of the compounding amounts of the hydroxyl group-containing monomer and the diketone group-containing monomer results in an additional improvement in performance.

On the other hand, as is apparent from Comparative Examples 1 to 4, when a pressure-sensitive adhesive layer is produced from an acrylic copolymer obtained without using one of the hydroxyl group-containing monomer and the diketone group-oontaining monomer, the gel fraction of the pressure-sensitive adhesive layer is low and stringiness becomes insufficient. Further, as is apparent from Comparative Examples 5 to 7, even when the hydroxyl group-containing monomer and the diketone group-containing monomer are used as copolymer components, in the case where these monomers are blended, the gel fraction of the pressure-sensitive adhesive layer is low and the stringiness is insufficient. The foregoing suggests that a proper three-dimensional network structure is not obtained even by blending a copolymer as a result of copolymerization involving using the hydroxyl group-containing monomer and a copolymer as a result of copolymerization involving using the diketone group-containing monomer, and the proper three-dimensional network structure is not formed until the hydroxyl group-containing monomer and the diketone group-containing monomer are copolymerized.

<Example 6>

An acrylic copolymer of the following formulation was obtained. 2-EHA: 85 parts, HEAA: 10 parts, AAEM: 5 parts

Further, a patch preparation was obtained in the same manner as in Example 1 except that: 5 parts of pramipexole (free base body) as a basic drug were added to 95 parts (solid content) of the acrylic copolymer; and IPM was not incorporated.

<Comparative Example 8>

A patch preparation was obtained in the same manner as in Example 6 except that an acrylic copolymer of the following formulation was used.
2-EHA: 75 parts, VP: 20 parts, AAEM: 5 parts

<Evaluation>

(4) Skin permeability (discharge property)

In order that a drug might be evaluated for its skin permeability, the drug was evaluated for its permeability from the skin of a hairless mouse (male, 8 weeks old) with a vertical membrane permeation test apparatus manufactured by VIDREX. The test temperature was 32˚C. A solution in a container was recovered at a predetermined time interval, and then the same amount of a 32˚C receptor liquid (physiological saline) was added to the solution. The operation was repeated, the cumulative amount ($\mu g/cm^2$) of the drug caused to permeate a certain area of the skin was calculated from the drug concentration (measured by HPLC) of the recovered solution, and the utilization ratio (%) of the drug was determined from the following equation.

$$\text{Utilization ratio } (\%) = \{\text{cumulative permeation amount } (\mu g/cm^2)/\text{drug content in preparation } (\mu g/cm^2)\} \times 100$$

As a result, the utilization ratio of the patch preparation of Example 6 after 24 hours was 77.3% and the utilization ratio of the patch preparation of Comparative Example 8 after 24 hours was 74.0%. That is, nearly about 80% of the drug in the patch preparation of Example 6 were discharged within 24 hours. Further, a significant difference in the discharge property of a drug was observed between the patch preparation of Example 6 and the patch preparation of Comparative Example 8.

The patch and patch preparation of the present invention can each be suitably utilized in, for example, the protection of a skin or the transdermal administration of a drug.

## Claims

**1.** A patch, comprising:

a support; and
a pressure-sensitive adhesive layer provided on at least one surface of the support,

wherein the pressure-sensitive adhesive layer contains an acrylic copolymer obtained by copolymerizing a monomer mixture containing (a) a (meth)acrylic acid alkyl ester, (b) a hydroxyl group-containing monomer, and (c) a diketone group-containing monomer.

**2.** A patch according to claim 1, wherein the pressure-sensitive adhesive layer contains an acrylic copolymer obtained by copolymerizing a monomer mixture containing 7.5 wt% to 20 wt% of the hydroxyl group-containing monomer (b).

**3.** A patch according to claim 1 or 2, wherein the pressure-sensitive adhesive layer contains an acrylic copolymer obtained by copolymerizing a monomer mixture containing 2.5 wt% to 7.5 wt% of the diketone group-containing monomer (c).

**4.** A patch according to any one of claims 1 to 3, wherein:

the pressure-sensitive adhesive layer further contains a plasticizer having compatibility with the acrylic copolymer; and
a weight ratio between the acrylic copolymer and the plasticizer is 1:0.1 to 1:2.

**5.** A patch according to any one of claims 1 to 4, wherein the hydroxyl group-containing monomer (b) comprises at least one selected from the group consisting of an N-hydroxyalkyl (meth)acrylamide and a hydroxyalkyl (meth) acrylate.

**6.** A patch according to claim 5, wherein the N-hydroxyalkyl (meth)acrylamide comprises at least one selected from the group consisting of an N-(2-hydroxyethyl)acrylamide and an N-(2-hydroxyethyl)methacrylamide.

**7.** A patch according to claim 5, wherein the hydroxyalkyl (meth)acrylate comprises a glycerin monomethacrylate.

**8.** A patch according to any one of claims 1 to 7, wherein the diketone group-containing monomer (c) comprises at least one selected from the group consisting of an acetoacetyl group-containing (meth) acrylic monomer and a diacetone acrylamide.

**9.** A patch according to any one of claims 1 to 8, wherein the pressure-sensitive adhesive layer has a gel fraction of 25 wt% to 70 wt%.

**10.** A patch according to any one of claims 1 to 9, wherein the acrylic copolymer self-cross-links to form a three-dimensional network structure without using any cross-linking agent.

**11.** A patch according to any one of claims 1 to 10, wherein the patch is a patch preparation having a drug incorporated

into the pressure-sensitive adhesive layer in the patch.

**12.** A patch according to claim 11, wherein the drug comprises a basic drug.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006064747 A
- JP 2005015536 A